# EUROPEAN PATENT APPLICATION

(11) **EP 1 785 096 A2**
(43) Date of publication of application: **16.05.2007**
(21) Application number: 06123955.4
(22) Date of filing: 13.11.2006
(51) Int. Cl.: A61B 10/06

(54) **DISPOSABLE BIOPSY PUNCH WITH SAMPLE RELEASING MECHANISM**

(30) Priority: 14.11.2005 US 735865 P
(71) Applicant: Kencap Ltd., Yahud 56101 (IL)
(72) Inventor: Porat, Amir, 56605, Savyon (IL); Porat, Agmon, 56605, Savyon (IL)
(74) Representative: Weydert, Robert

(57) **Abstract**

A disposable biopsy punch (15), comprising a first shaft (18) and a second shaft (20) pivotally connected at a pivot point (23). Each shaft terminates in a jaw (30,32) having a cutting tooth (36,40). A first shaft is characterized by having a plurality of guides (26,28,44) projecting therefrom, and the second shaft has a plurality of orifices (42,46,48) mating with the guides upon closure of the punch, to provide stabilization of the punch. The punch also comprises a sample releasing mechanism (34).

## Description

### FIELD OF THE INVENTION

The invention relates generally to surgical instruments, and more particularly to a disposable biopsy punch.

### BACKGROUND OF THE INVENTION

When an abnormality is suspected in the cervix, a biopsy is typically obtained using a biopsy forceps commonly known as a biopsy punch. The tissue-collecting end of the punch is introduced through a speculum into the vagina, to reach the cervix. Commonly used biopsy punches often resemble forceps, having a pair of bows serving as handles, which accommodate the finger and thumb, and a lengthened shaft ending in a pair of jaws which can be closed upon the tissue to sever it. Most often, the upper jaw is sharpened and can be actuated to move upon its axis when pressure is applied on the handles. The lower jaw is stationary and contains a hollowed indent into which the severed tissue will fall. The jaws remain closed over the tissue, and the punch is removed from the vagina, removing the tissue sample within them. A typical prior art biopsy punch is shown in U.S. Patent No. 5,643,307, Figure 1.

Biopsy punches are made of metals such as stainless steel, and must be cleaned and sterilized between each use to prevent cross-contamination between patients. The sterilization process is time-consuming and cost-generating, and cannot promise absolute sterility if performed in an ineffective manner.

A disposable biopsy punch trademarked Unigyn® has recently been manufactured by Gyn Instruments Ltd., Nice, France. The punch resembles a scissor-like forceps having two shafts connected at a hinge, and both shafts along with their respective jaws, move towards one another when the handles are pressed together. Since the punch is made of polycarbonate, which is semi-flexible at the thickness used for a punch, the shafts must be prevented from buckling and moving laterally when the handles are brought together to close the jaws upon the tissue. Lengthened guides are present for this purpose, projecting from one of the shafts. When the handles are pressed to bring the shafts closer together and close the jaws, each of the guides enters an appropriate hole in the far shaft, and the punch shafts are stabilized. The punch suffers the disadvantage of having an overly large diameter, due to the lengthened guides projecting from out of the far side of their holes. These guides prevent insertion of the forceps deep into the vagina to reach the cervix, since the projecting guides will not fit through an opened speculum. The forceps can therefore only be used to reach an area located at a shallow depth within the vagina, since only the tip of the forceps can enter the vagina.

After collection of a sample within the jaws of a punch, and retraction of the punch from the vagina, the sample needs to be removed from the hollowed area within the jaws of the punch, for analysis. The removal is accomplished either using a tweezers, which pinches the sample and may partially damage the sample, or by dipping the punch into a fluid-filled vessel, to float the sample out of the hollowed area. The sample is then fished from the fluid using a tweezers. It would be advantageous to provide a simpler method for removal of the sample from within the punch jaws.

The need exists for a disposable biopsy punch that can be used to collect a cervical biopsy from any depth within the cervix; such punch needs to be durable and not buckle upon use. Such punch should be discarded after a single use, thus eliminating the cleansing and sterilization process. The need exists for a sample releasing mechanism provided within said punch.

### SUMMARY OF THE INVENTION

It is the object of the invention to overcome those drawbacks found in the prior art and to provide a disposable punch useful for taking a biopsy, comprising: a first shaft and a second shaft pivotally connected at a pivot point. Each of said shafts terminates in a jaw having a cutting tooth, and each shaft has a handle at its proximal end for accommodating a finger or thumb. The first shaft is characterized by having a plurality of shortened guides projecting from the shaft. The second shaft has a plurality of orifices mating with said shortened guides upon closure of the punch. The guides and orifices mate to provide stabilization of the punch. The punch comprises a sample releasing mechanism.

In the present invention, the punch is described for use in removing a biopsy from the female gynecological tract. This description is for illustrative purposes only, and is not intended to limit use of the punch to this area of the human body. One can envision use of the punch in collecting a biopsy from any other area of the human or mammalian body.

These and other advantages will become apparent from the detailed description that follows.

### BRIEF DESCRIPTION OF THE DRAWINGS

For a better understanding of the invention, with regard to the embodiments described, reference is made to the accompanying drawings, in which like numbers designate corresponding elements or sections throughout, and in which:
Fig. 1 is a side view of a closed punch;
Fig. 2 is a perspective view showing an enlargement of the jaws and shafts of the punch when opened;
Fig. 3 is a side view of the punch with directional arrows showing direction of pulling on the shafts for easy removal of the sample;
Fig. 4 is a side view showing pivoted shafts for removal of the sample;
Fig. 5 is a side view showing shafts at their most extreme position for removal of the sample;
Fig. 6 is an enlarged perspective view showing the jaws including the slotted base of the hollowed area;
Fig. 7 is an enlarged perspective view showing the jaws after the sample release mechanism has been used to remove the sample from the punch; and:
Fig. 8 is a perspective view showing the entire punch after the sample release mechanism has been used to remove the sample from the punch.

### DETAILED DESCRIPTION OF THE PREFERRED EMBODIMENTS

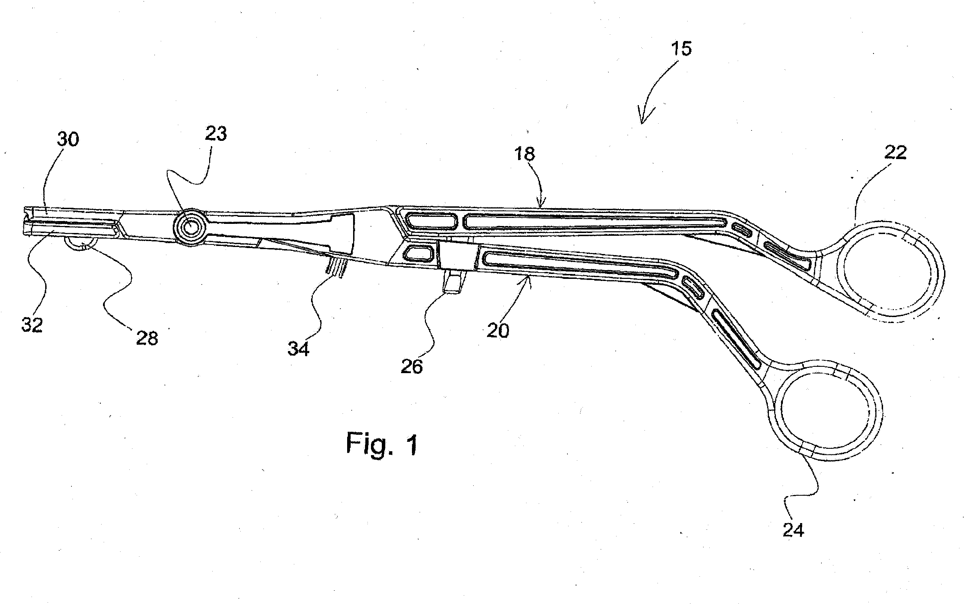

The disposable biopsy punch of the invention is shown generally in Figure 1. The punch 15 is comprised of a first shaft 18 and a second shaft 20, pivotally connected at pivot point 23. Each shaft has a handle or bow 22, 24 at its proximal end for accommodating a finger or thumb, and each shaft terminates in a jaw 30, 32 having a sharpened cutting tooth for removal of a sample. The punch 15 is shown in Figure 1 in its closed state, with shafts 18, 20 in close proximity to each other.

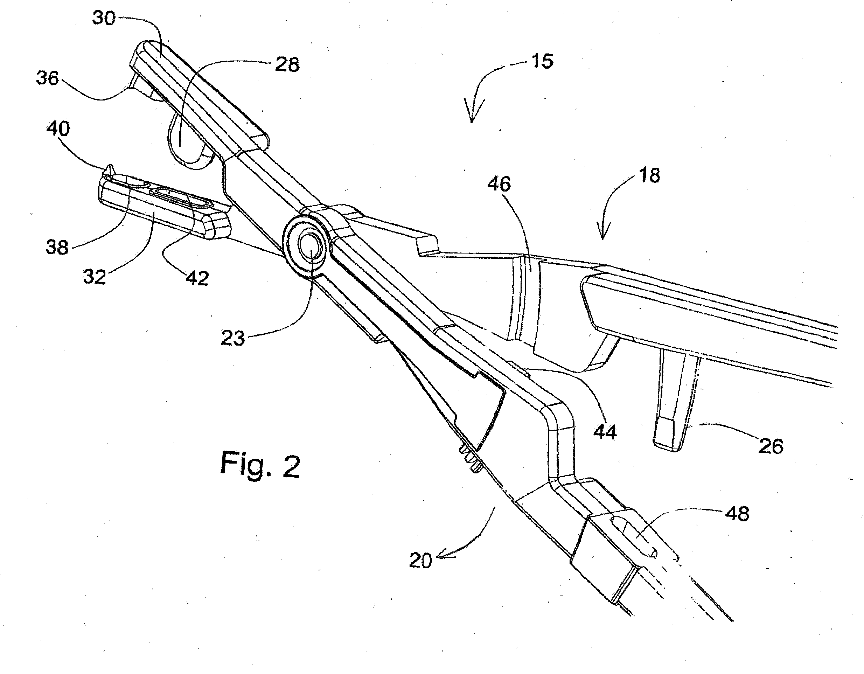

The punch 15 is preferably made of polycarbonate. Alternatively, the punch can be formed of delarin, nylon, or similar materials. Preferably, the punch is reinforced with glass fibers. Since these materials are semi-flexible at the thickness used for a punch, the shafts must therefore be prevented from buckling and moving laterally when the handles are brought together to close the jaws upon the tissue. Guides 26, 28 act to stabilize the shafts 18, 20 and prevent lateral movement of the shafts 18, 20 when the user presses on the handles 22, 24 in order to bring the shafts 18, 20 in proximity to one another and close the jaws 30, 32 upon the tissue to take a biopsy sample. Shortened guides 26, 28 which extend from the first shaft 18, have mated with and are seen projecting from the far side of appropriate orifices present on second shaft 20. These orifices are best shown in Figure 2.

The shortened guides 26, 28 are of such length that they do not overly project to hinder insertion of the punch past a speculum into the vagina. For instance, the length of the guides may be within the approximate range of 0.3 cm to 1 cm, so that upon closure of the shafts together, the entire width of the punch when measured along the longest guide, will be close to 3 cm. This width can easily pass through a speculum into the vagina.

A plurality of fingers 34 project from one of the shafts 18, 20. Fingers 34 act as part of a mechanism for removal of the sample from within the punch 15. This mechanism will be described hereinbelow.

Referring to Figure 2, there is shown an enlarged view of the punch with the shafts 18, 20 opened. Handles for accommodating finger and thumb are not shown in this Figure. Sharpened tooth 36 protrudes from jaw 30, and can enter hollowed area 38 when the punch 15 is closed upon the tissue. Opposing sharpened tooth 40 will close beyond tooth 36 and both teeth act to sever the tissue. Tissue sample will remain entrapped within hollowed area 38.

Optionally, one or both teeth 36, 40, can be replaced with a metal ring which will perform the cutting. Such a ring should have a diameter of 3-5 mm, and may be elliptical in shape. Alternatively, a metal insert plate or a metal covering (not shown) can be present upon any the inner surfaces of the jaw or upon the teeth, for providing optimal cutting capabilities. The jaws or selected areas of the jaws may be manufactured of metal. In such case, the material used to manufacture the punch need not be reinforced with glass fibers, as the metal segments will provide sufficient strength for cutting of a sample.

Upon closure of the jaws 30, 32, guide 28 will mate with orifice 42 and stabilize the jaws 30, 32 in proper alignment with one another. Guide 44 enters recess 46 to contribute to the stabilization, and guide 26 mates with and enters orifice 48.

After the sample has been taken and the punch has been removed from the human body with the sample enclosed within the hollowed area 38, the sample can be easily removed from within the hollowed area using the sample releasing mechanism, as described hereinbeleow.

According to one embodiment, the dimensions of the hollowed area 38 are 2mm wide and 7mm long. Optionally, the hollowed area can be rounded (not shown), having a diameter of 3-5 mm.

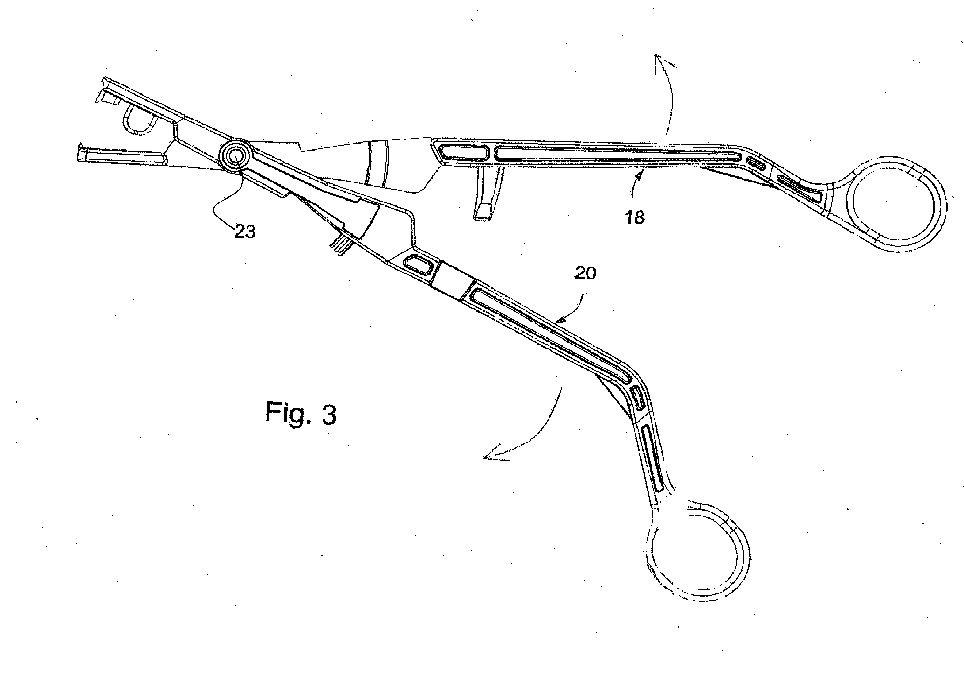

Referring now to Figure 3, there is shown a side view of the punch, with shafts open relative to one another. To easily remove the sample from the punch, shafts 18, 20 are pulled apart in the direction shown by the arrows.

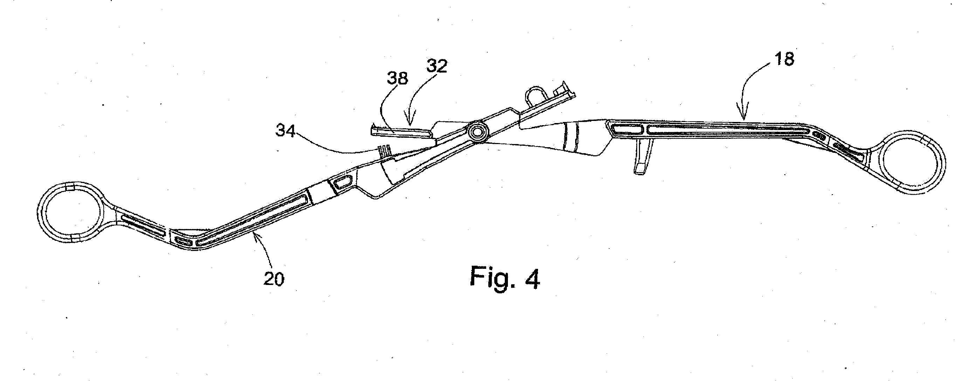

Referring to Figure 4, shafts 18, 20 are pivoted upon pivot point 23, until the shafts are at an approximate angle of 180° relative to one another. Fingers 34 are now aligned with hollowed area 38 present within jaw 32. The base, or lowermost portion of hollowed area 38, is slotted, and mates with fingers 34.

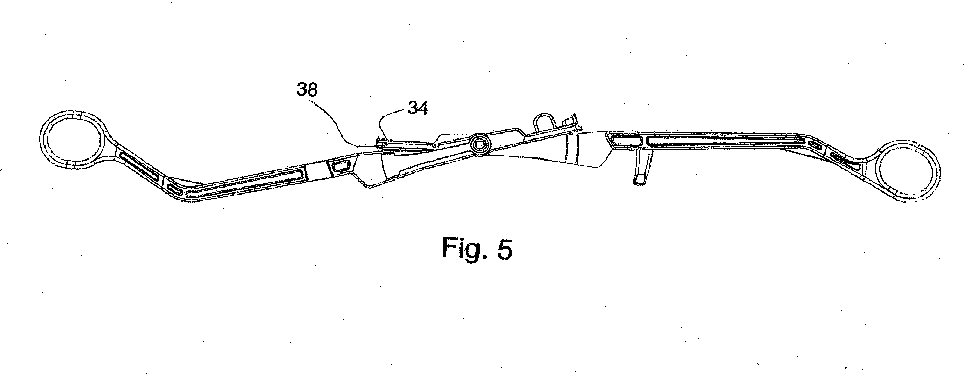

Referring to Figure 5, when the shafts are locked open at their most extreme position, fingers 34 enter slotted base of hollowed area 38 to fill the majority of the hollowed area and drive the sample out from the hollowed area 38.

The sample release mechanism thus allows the sample to be pushed out of the jaws of the punch, in a rapid manner in which success is guaranteed. The mechanism is hassle-free, especially when compared to the difficult prior art method of sample removal.

Referring back to Fig. 4, in an alternative embodiment of the sample release mechanism, a segment of shaft 20 from which fingers 34 project, is removable from the punch. After a sample has been taken and is enclosed within hollowed area 38, this shaft segment is removed from the punch, the jaws are opened, and the fingers 34 are inserted into slots of slotted base from below shaft 18, to drive sample upwards and out of hollowed area 38. In this embodiment, shafts need not be pivoted 180° upon their pivot point to release the sample. Removal of the fingers 34 for use in sample release may be accomplished by the user by breaking the fingers 34 along a pre-weakened breakage line. The fingers 34 may be removed with or without a segment of the shaft 20. Alternatively, the fingers may be provided with other forms of attachment to the shaft, such that allow straightforward removal of the fingers after the sample is taken.

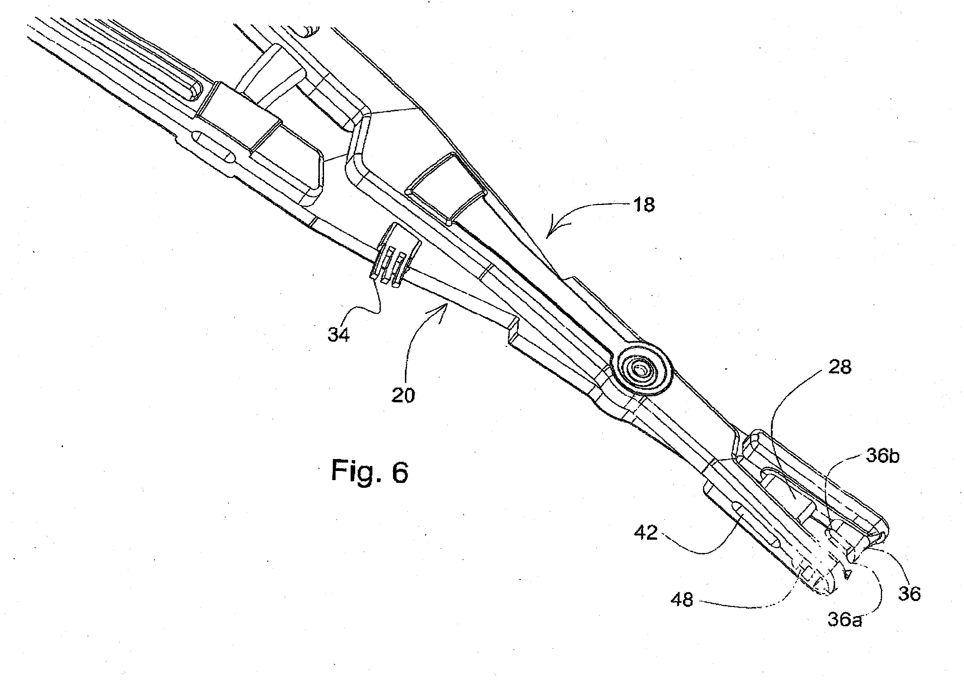

Referring to Figure 6, an enlargement is shown of the jaws of the punch, when the jaws are in position to take a biopsy sample. Slotted base 48 of hollowed area 38 is shown from below. Fig. 6 shows orifice 42, which mates with guide 28. Note the shape of tooth 36, which has sharpened portion 36a projecting upwards to cut, and support 36b which has an elevated rim to grasp the sample when enclosed within the jaws, ready for removal of the punch from the vagina. Fingers 34 are seen in this enlarged view.

Optionally, slotted base 48 of the hollowed area 38, and/or upper support 36b, or other areas of the jaws, may be reinforced with a metal plate present within (not shown) or a metal covering, to provide additional physical strength to these areas of the punch.

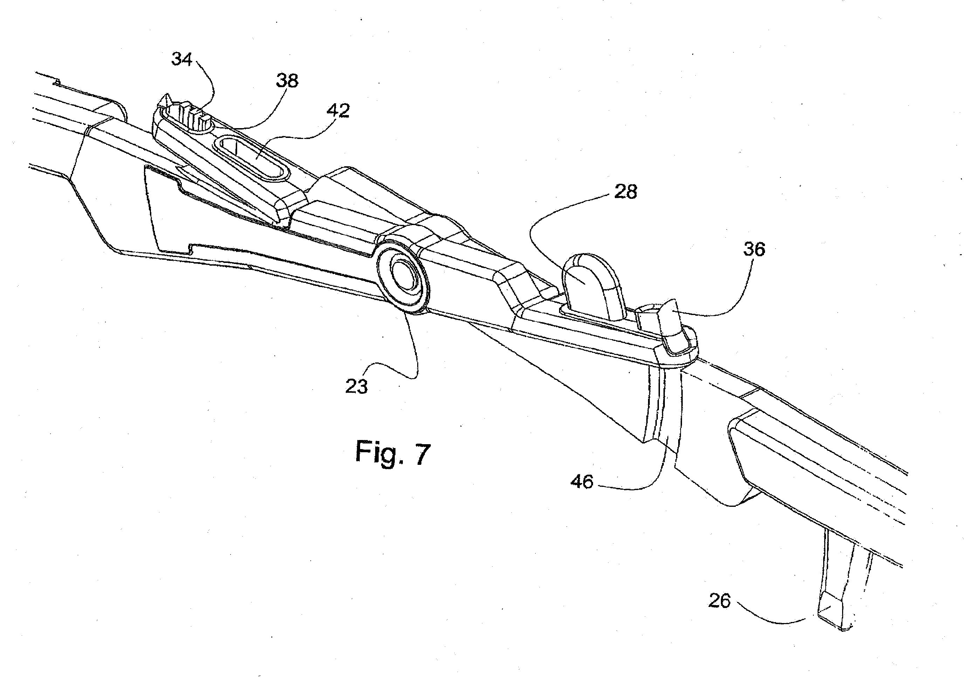

Referring to Figure 7, an enlargement is shown of the jaws, after the sample release mechanism has been used to remove the sample from the punch. Fingers 34 have mated with the slotted base of the hollowed area 38, and protrude from the hollow 38, thus essentially pushing the sample from the hollow. Note the raised rim present around hollowed area 38, and around orifice 42 (guide 28 mates with orifice 42). The raised rim ensures the sample within the hollow will not be crushed when the punch is closed over the sample and the punch is retracted from the body.

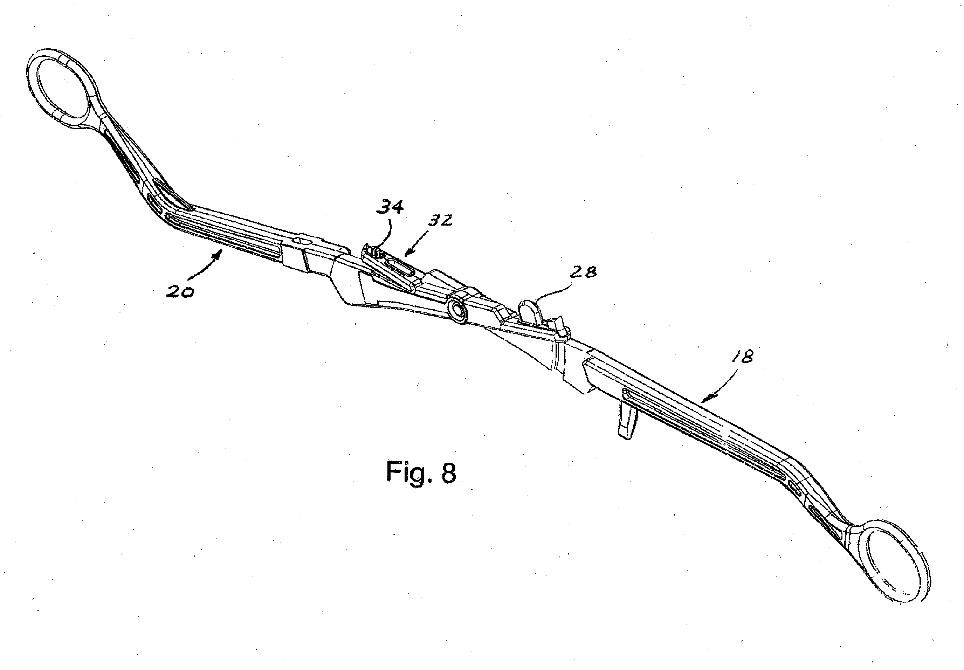

Figure 8 is a partial side view of the punch after the sample release mechanism has been used.

Having described the invention with regard to certain specific embodiments thereof, it is to be understood that the description is not meant as a limitation, as further modifications will now become apparent to those skilled in the art, and it is intended to cover such modifications as are within the scope of the appended claims.

## Claims

1. A disposable biopsy punch, comprising:
a first shaft and a second shaft pivotally connected at a pivot point, each of said shafts terminating in a jaw having a cutting tooth,
each shaft having a handle at its proximal end for accommodating a finger or thumb,
wherein said first shaft is **characterized by** having a plurality of shortened guides projecting from said shaft,
said second shaft having a plurality of orifices mating with said shortened guides upon closure of said punch, said guides and orifices mating to provide stabilization of said punch,
said punch further comprising a sample releasing mechanism.

2. The punch of claim 1, wherein said sample releasing mechanism is provided by a plurality of fingers projecting from one of said shafts, wherein the other of said shafts has a hollowed area with a slotted base within the jaw thereof,
said shafts being pivotable 180° about their pivot point, such that after pivoting of said shafts 180° about their pivot point, said fingers can mate with the slots of said slotted base to fill the majority of said hollowed area and drive a sample out from said hollowed area.

3. The punch of claim 1, wherein said sample releasing mechanism is provided by a plurality of fingers projecting from a removable portion of one of said shafts, wherein the other of said shafts has a hollowed area with a slotted base within the jaw thereof,
wherein after removal of said removable portion of said one of said shafts, said fingers can be aligned beneath said other shaft, to mate with the slots of said slotted base to fill the majority of said hollowed area and drive a sample out from said hollowed area.

4. The punch of claim 1, wherein said punch is manufactured from a material selected from: semi-flexible polycarbonate, nylon and delarin.

5. The punch of claim 4, wherein said material is reinforced with glass fibers.

6. The punch of claim 1, wherein the length of said shortened guides is such as to allow insertion of the punch through an opened speculum into a cervix.

7. The punch of claim 1, wherein the length of said shortened guides is within the approximate range of 0.3 cm to 1 cm, and wherein upon closure of said shafts together, the width of said punch when measured along the longest guide, will be approximately 3 cm.

8. The punch of claim 1, wherein one of said cutting teeth has a support with an elevated rim to grasp a sample when enclosed within said jaws, during removal of the punch from the human body.

9. The punch of claim 2, wherein at least one of said hollowed area and said orifices is surrounded by a raised rim to ensure a sample will not be crushed when the punch is closed over said sample and the punch is retracted from the human body.

10. The punch of claim 2, wherein the dimensions of said hollowed area are within the range of 2-7mm.

11. The punch of claim 1, wherein at least one portion of said jaws of said punch is manufactured of metal or coated with metal, for providing optimal cutting capabilities to said jaws.
